# EUROPEAN PATENT APPLICATION

(11) **EP 0 808 902 A2**
(43) Date of publication of application: **26.11.1997**
(21) Application number: 96307159.2
(22) Date of filing: 30.09.1996
(51) Int. Cl.: C12N 15/29, C07K 14/415, C12N 1/21

(54) **Novel genes encoding antiviral proteins of Phytolacca insularis Nakai and recombinant microorganisms expressing the same proteins**

(30) Priority: 22.05.1996 KR 9617404
(71) Applicant: JINRO LIMITED, Seoul (KR)
(72) Inventor: Moon, Young-Ho, Kyonggi-Do (KR); Choi, Jin-Nam, Kyonggi-Do (KR); Yun, Young-Chae, Suwon, Kyonggi-Do (KR); Jin, Jung-Hun, Suwon, Kyonggi-Do (KR); Hong, Eun-Ju, Kyonggi-Do (KR); Lee, Jeong-Ho, Yongin, Kyonggi-Do (KR); Choi, Kyu-Whan, Pochun-Kun, Kyonggi-Do (KR); Lee, Jong-Seob, Seocho-Ku, Seoul (KR); Song, Sang-Kee, Seoul (KR); Choi, Yang-Do, Seoul (KR); Kim, Chul-Hwan, Seoul (KR); Kim, Man-Keun, Seoul (KR)
(74) Representative: Woods, Geoffrey Corlett

(57) **Abstract**

Genes coding for antiviral proteins isolated from genomic DNA and a genomic library of Phytolacca insularis Nakai, and antiviral proteins having the amino acid sequences deduced from the said genes, expression vectors containing the said genes, and processes for preparing the antiviral proteins from recombinant microorganisms transformed with the said vectors. The antiviral proteins can be used as an active ingredient in plant antiviral agents and can be employed in immunoconjugates for the treatments of AIDS and cancer.

## Description

### Field of the Invention

The present invention relates to novel genes coding for antiviral proteins of Phytolacca insularis Nakai and recombinant microorganisms expressing the proteins, more specifically, novel genes coding for antiviral proteins isolated from genomic library of Phytolacca insularis Nakai, antiviral proteins having the amino acid sequences deduced from the said genes, expression vectors containing the said genes, and processes for preparing the antiviral proteins from recombinant microorganisms transformed with the said vectors.

### Background of the Invention

Since plants can not escape from foreign pathogenic organisms due to their immobile nature, they must be able to defend themselves by making direct or indirect response to the pathogenic challenge; and, most plants appear to undertake some general defense mechanism to protect themselves against infective pathogens, e.g., fungi, bacteria and viruses.

In this connection, studies on the antiviral proteins from many different plant species, have been carried out, starting from the discovery of pokeweed antiviral protein(PAP) isolated from crude extract of Phytolacca americana L. (see: Irvin, J.D., Arch. Biochem. Biophys., 169:522-528(1975)). In addition to the PAP, antiviral proteins have been successively isolated from several plants, e.g., Ricin(from Ricinus communis)(see: Halling, K.C. et al., Nucleic Acid Res., 13:8019-8033(1985)), Mirabilis antiviral protein(from Mirabilis jalapa L.)(see: Kataoka, J. et al., J. Biol. Chem., 266:8426-8430(1991)), and α-trichosanthin(from Trichosanthes kirilowii)(see: Zhang, X. et al., Nature, 321:477-478(1986)).

It has been also reported that said antiviral proteins are fallen within a group of ribosome inactivating protein (RIP) and have RNA N-glycosidase activities, by which impair ribosomes by removing adenine in the specific region of rRNA and inhibit the synthesis of proteins(see: Stirpe, F. et al., Bio/Technology, 10:405-412(1992)). As for the survival mechanism of the plants producing RIP without being influenced by their own RIP having those activities, it has been suggested that the plants synthesize ribosomes resistant to their own RIP, or immature RIPs are moved out from the plant cell, before the maturation of RIP, through endoplasmic reticula and Golgi bodies by the aid of signal peptide .

While RIP has been known to give plant an antiviral resistance, its mode of action has not been clearly understood yet, except for a fact that RIP located out of a plant cell enters the cell together with infecting virus, kills the infected cell, which, in turn, inhibits the infection and spread of the virus(see: Fernandez-Puentes, C. et al., Cell, 20:766-775(1980)).

Anyway, antiviral property of RIP has prompted the use of RIP in the manufacture of transgenic plants resistant to virus and immunoconjugates for the treatment of AIDS or cancer(see: Lodge, J.K. et al., Proc. Natl. Acad. Sci., USA, 90:7089-7093(1993); Ramarkrishnan, S.D. et al., Ann. Rev. Pharmacol. Toxicol., 32:579-621(1992); Taylor, S.A. et al., Plant J., 5:827-835(1994)). Specifically, considering a fact that there is no remarkable specific remedy for great damage of crops by viral infection, there has been strong reasons for exploring and developing transgenic plants having resistance to pathogenic viruses.

In line with these activities, the present inventors have deeply studied antiviral proteins of various plants including Phytolacca americana L., Phytolacca insularis Nakai, Cucurbita moschata, and so on, and isolated novel genes encoding their antiviral proteins finally to give the desired antiviral transgenic plants transformed with the recombinant expression vectors containing the said genes (see: USP 5,348,865; Japanese patent 2522151; and, Australian patent 663031).

### Summary of the Invention

In accordance with the present invention, the present inventors have isolated novel genes coding for antiviral protein from a genomic library of Phytolacca insularis Nakai, and discovered that all of the recombinant proteins produced from the expression vectors containing said genes are novel antiviral proteins.

A primary object of the present invention is, therefore, to provide novel genes coding for antiviral proteins of Phytolacca insularis Nakai, and novel antiviral proteins having amino acid sequences translated therefrom.

The other object of the invention is to provide expression vectors comprising the said genes and recombinant microorganisms transformed with the vectors.

Another object of the invention is to provide processes for preparing recombinant antiviral proteins from the said microorganisms.

### Brief Description of Drawings

The above and the other objects and features of the present invention will become apparent from the following description given in conjunction with the accompanying drawings, in which:
- Figure 1: is the nucleotide sequence of gPIP2 gene and amino acid sequence deduced therefrom (SEQ ID NO:1 and SEQ ID NO:2);
- Figure 2: shows a construction strategy of an expression vector comprising the gPIP2 gene isolated from a genomic library of Phytolacca insularis Nakai;
- Figure 3: is a photograph showing agarose gel electrophoresis pattern of expression vectors comprising gPIP2 or gPIP50 gene digested with restriction enzyme;
- Figure 4: is a photograph showing agarose gel electrophoresis pattern and Southern hybridization of phage DNA which is isolated from clone #50 in a genomic library of Phytolacca insularis Nakai and digested with SacI or XhoI;
- Figure 5: is the nucleotide sequence of gPIP50 gene and amino acid sequence translated therefrom(SEQ ID NO:3 and SEQ ID NO:4);
- Figure 6: shows a stepwise construction strategy of an expression vector comprising the gPIP50 gene derived from plasmid pPIP50;
- Figure 7: is a photograph showing SDS-PAGE pattern of total protein of inclusion body, pure recombinant gPIP2 and gPIP50 proteins, and supernatant obtained by homogenizing E. coli transformed with the expression vectors containing gPIP2 or gPIP50 genes; and,
- Figure 8: is an autoradiogram showing the inhibition of protein synthesis occurring when in vitro translation assay of gPIP2 or gPIP50 purified from the recombinant E. coli is carried out.

### Detailed Description of the Invention

The present inventors first isolated a genomic DNA from leaves of Phytolacca insularis Nakai by the method conventionally used in the art(see: J. Sambrook et al., Molecular Cloning, A Laboratory Manual, Cold Spring Harbor Laboratory, 1989) and performed polymerase chain reaction(PCR) by employing the genomic DNA as a template, 5'-CCAAGCTTGTGAATACCATCATCTAC-3'(SEQ ID No:5) as NH₂-terminal primer, and 5'-GGAAGCTTTGATCAGAAACCCTCAAA-3'(SEQ ID No:6) as COOH-terminal primer, respectively. 1kb-DNA fragment thus amplified was digested with HindIII, and cloned on pUC19 vector. And then, its restriction map was prepared and nucleotide sequencing was followed. As a result, the amplified DNA fragment was found to comprise a coding region of mature protein which consists of 882bp including initiation codon of translation, and it was designated 'gPIP2 gene'. Also, open reading frame of gPIP2 gene was revealed to code for a 32.8kDa-protein which consists of 292 amino acids including initiation methionine, and the protein was designated 'gPIP2 protein'.

Based on the comparison study on the amino acid sequence deduced from the said gPIP2 gene with the ones from cPAP and cPIP genes coding for antiviral proteins (see: Lin et al., Plant Molecular Biology, 17(4):609-614(1991); USP 5,348,865), one from the gPIP2 gene was found to show high level of homology to them, i.e., 93.1% and 82.5% homology to ones from cPAP and cPIP genes, respectively. Therefore, it was suggested that the gPIP2 protein is a novel antiviral protein whose activity is very similar to the antiviral proteins translated from the cPAP and cPIP genes but whose amino acid sequence is somewhat different from them. Further, comparison of the nucleotide sequence of the gPIP2 gene with ones of various antiviral protein genes revealed that the gPIP2 gene is novel one.

On the other hand, the inventors have prepared genomic library based on the genomic DNA from leaves of Phytolacca insularis Nakai, and selected nine positive plaques using the cPIP gene labelled with [α-³²P]dATP as a DNA probe. Then, phage DNAs were isolated from those plaques, digested with SacI or XhoI, and analyzed by Southern hybridization using the same DNA probe. As a result, clone #50 in which clear hybridization occurred at the bands of 5.0kb XhoI fragment, and two 4.8kb- and 3.5kb- SacI fragments, was finally selected.

5.0kb-XhoI fragment from the selected clone #50 was digested with various restriction enzymes, and analyzed by Southern hybridization. As a result, one positive signal appeared at the band of 3.4kb-BamHI DNA fragment. Thus, the said 3.4kb-BamHI fragment was subcloned on pUC19 vector, and its restriction map was prepared and its nucleotide sequence was determined. The gene was found to comprise a coding region of mature protein which consists of 951bp including initiation codon of translation, and designated 'gPIP50 gene'. Also, open reading frame of gPIP50 gene was revealed to code for a 35.7kDa-protein which consists of 315 amino acids including initiation methionine, and the protein was designated 'gPIP50 protein'.

Based on the comparison of the amino acid sequence deduced from the said gPIP50 gene with the known antiviral proteins translated from cPIP or α-PAP gene(see: USP 5,348,865; J. Kataoka et al., Plant Mol. Biol., 20:879-886(1992)), one from the gPIP50 gene was found to show high level of homology to them, i.e., 69.4% and 83% homology to ones from cPIP and α-PAP genes, respectively. Therefore, it was suggested that the gPIP50 protein is a novel protein which may have an antiviral activity. Further, comparison of the nucleotide sequence of the gPIP50 gene with ones of various antiviral protein genes, revealed that the gPIP50 gene is novel one.

The inventors have manufactured a recombinant expression vector by inserting the isolated gPIP2 or gPIP50 gene into a vector which comprises FLAG sequence of octapeptide participating in binding with an anti-FLAG monoclonal antibody and cleavage with enterokinase, and multiple cloning sites participating in insertion of coding sequence, and then, transformed a competent E. coli with the said vector, cultured the transformant, and induced the expression of the recombinant gPIP2 or gPIP50 protein. Cultured transformants were ultrasonicated, and the expression of the recombinant gPIP2 or gPIP50 protein was investigated. As a result, it was found that the said protein was successfully produced from the transformants in a form of inclusion body.

In order to confirm whether the recombinant gPIP2 and gPIP50 proteins inhibit the protein synthesis like known antiviral proteins, the recombinant gPIP2 and gPIP50 proteins were purified from total proteins obtained from the inclusion body of the said transformant and in vitro translation assay was carried out, which finally confirms that they successfully inhibit the protein synthesis.

Accordingly, it was clearly determined that the gPIP2 and gPIP50 genes isolated from Phytolacca insularis Nakai, can be employed in the manufacture of transgenic plants having antiviral resistance. Also, the recombinant gPIP2 and gPIP50 proteins prepared from the transformed microorganisms harboring expression vectors containing gPIP2 and gPIP50 genes can be used as an active ingredient of antiviral agents of plant viruses, and employed in the manufacture of immunoconjugates for the treatment of AIDS and cancer.

In describing the amino acid sequence of the present invention, the term 'proteins consisting of functionally equivalent amino acids' is employed to mean all proteins substituted by the combinations such as Gly, Ala; Val, Ile, Leu; Asp, Glu; Asn, Gln; Ser, Thr; Lys, Arg; and, Phe, Tyr among the amino acid sequences of gPIP2 or gPIP50 protein, and in describing the nucleotide sequence of the invention, the term 'functional equivalents' means all genes comprising nucleotide sequences coding for all the said combinations.

The present invention is further illustrated by the following examples, which should not be taken to limit the scope of the invention.

### Example 1: Isolation of gPIP2 gene from genomic DNA of Phytolacca insularis Nakai and construction of an expression vector containing the gene

Genomic DNA was isolated from leaves of Phytolacca insularis Nakai, and gPIP2 gene was amplified by PCR using the gene as a template. Then, the amplified gPIP2 gene was inserted into pFLAG-1™ vector to construct an expression vector for the gPIP2 protein.

### Example 1-1: Isolation of genomic DNA of Phytolacca insularis Nakai

Genomic DNA was isolated from leaves of Phytolacca insularis Nakai in accordance with Shure et al's method(see: Shure, M., et al., Cell, 35:225-233(1983)): 1g of leaves of Phytolacca insularis Nakai was grinded under a stream of liquid nitrogen, mixed well with the addition of 5ml of DNA extraction buffer, and digested with 250µl of proteinase K solution(5mg/ml) for 15 minutes at 65°C. Then, phenol/chloroform extraction and centrifugation were carried out to obtain the supernatant.

The same volume of phenol/chloroform/isoamylalcohol mixture(25:24:1(v/v/v)) was added to the said supernatant and centrifuged to obtain the supernatant. Then, NaCl and 10% CTAB(cetyltrimethylammonium bromide) were added to the supernatant thus obtained to a final concentration of 1.5M and to reach 1/10 of total volume of the mixture, respectively, and incubated for 5 minutes at 65°C. Two cycles of chloroform extraction were followed to obtain supernatant, and 0.5ml of 4.4M NH₄OAc(pH 5.2) per 1g of sample and the same volume of isopropylalcohol as the supernatant were added and mixed slowly.

Then, the precipitated genomic DNA was isolated, dissolved in 0.75ml of TE buffer(10mM Tris-HCl buffer(pH 8.0) containing 1mM EDTA), incubated for 30 minutes at 60°C with the addition of RNase A to a final concentration of 10µg/ml, and extracted with phenol/chloroform as described aboves. After the addition of NH₄OAc(pH 5.2) to a final concentration of 0.6M and the same volume of isopropylalcohol as the supernatant, the genomic DNA was isolated.

### Example 1-2: Isolation of a gPIP2 gene and determination of the nucleotide sequence of the gPIP2 gene

PCR was performed using the genomic DNA obtained in Example 1-1 as the template, and oligonucleotides as followings as primers:
NH₂-terminal primer
5'-CCAAGCTTGTGAATACCATCATCTAC-3'(SEQ ID No:5)
COOH-terminal primer
5'-GGAAGCTTTGATCAGAAACCCTCAAA-3'(SEQ ID No:6)
That is, 1µg of the genomic DNA of Phytolacca insularis Nakai and each 200ng of the primers were mixed and denaturated for 5 minutes at 95°C. Then, denaturation(95°C, 1 minute), annealing(55°C, 1 minute), and extension(72°C, 1 minute) were carried out for 30 cycles, respectively, employing DNA Thermal Cycler(Cetus/Perkin-Elmer, USA) where the gene was amplified using Vent™ DNA polymerase(NEB, USA).

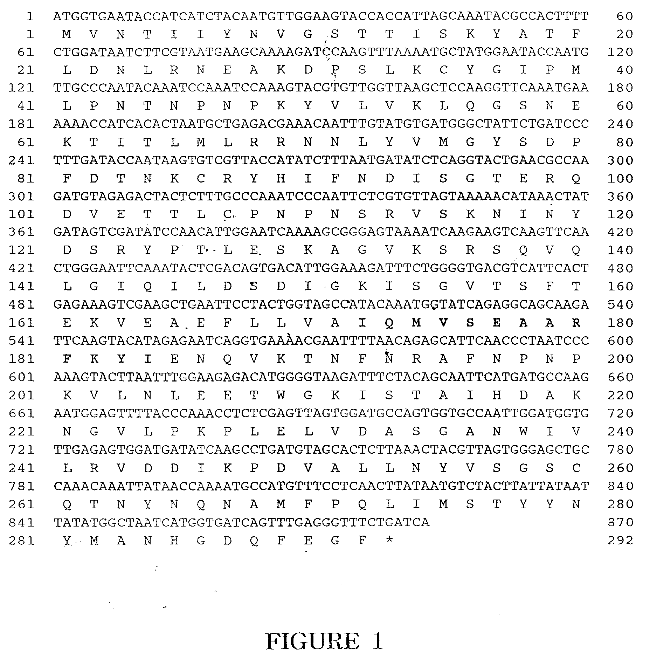

Amplified 1kb-DNA fragment containing the gPIP2 gene was cleaved with HindIII and cloned on pUC19 vector, and restriction enzyme map was prepared. To determine the full nucleotide sequence of the gPIP2 gene, the gPIP2 gene was cleaved with EcoRI or XhoI, and deletion series were made by Erase-a-Base system(Promega, USA). Then, the nucleotide sequences of the deletion series were determined by the dideoxy nucleotide chain termination method (see: Sanger, F. et al., Proc. Natl. Acad. Sci., USA, 74:5463-5467(1977)) employing Sequenase™(United States Biochemical Co., USA). As a result, it was revealed that the gPIP2 gene coding for the mature protein consists of 882bp including the start codon of translation, and its open reading frame encodes a 32.8kDa-protein which consists of 292 amino acids including initiation methionine(see: Figure 1). Figure 1 shows the nucleotide sequence of the gPIP2 gene and amino acid sequence deduced therefrom(SEQ ID NO:1 and SEQ ID NO:2). In Figure 1, amino acid sequence in bold letter represents one involved in putative active site, and asterisk(*) represents a termination site of translation, respectively.

Based on the comparison of the amino acid sequence deduced from the said gPIP2 gene with one from cPAP or cPIP gene coding for a known antiviral protein, one from the gPIP2 gene was found to show high level of homology to them, i.e., 93.1% homology to one from cPAP gene, and 82.5% homology to one from cPIP gene. Therefore, it was suggested that the gPIP2 protein may possess an antiviral activity very similar to the antiviral proteins translated from the cPAP and cPIP genes.

### Example 1-3: Construction of an expression vector for the gPIP2 gene

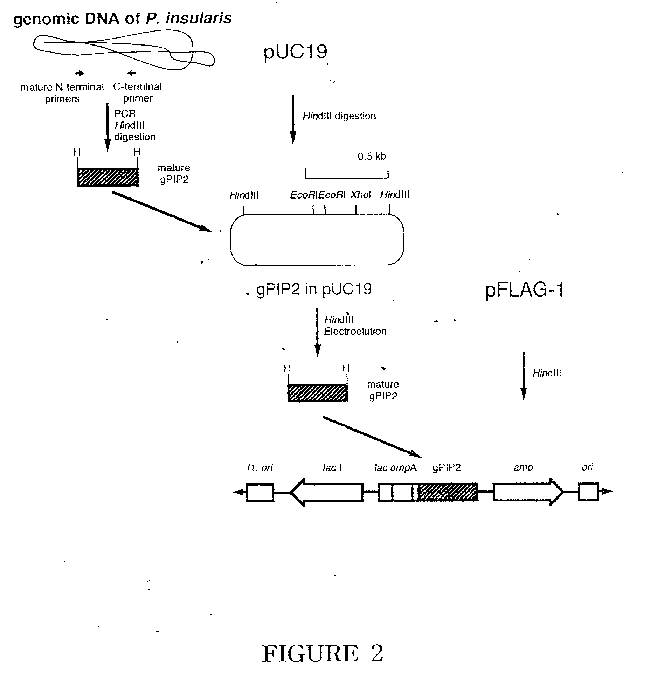

1kb-DNA fragment amplified in Example 1-2 was digested with HindIII, and inserted into a pFLAG-1™ vector (International Biotechnologies Inc., USA) which comprises FLAG sequence of octapeptide participating in binding with an anti-FLAG monoclonal antibody and cleavage of enterokinase, and multiple cloning sites participating in insertion of coding sequence (see: Figure 2). Figure 2 shows a construction strategy of an expression vector comprising a gPIP2 gene isolated from a genomic DNA of Phytolacca insularis Nakai.

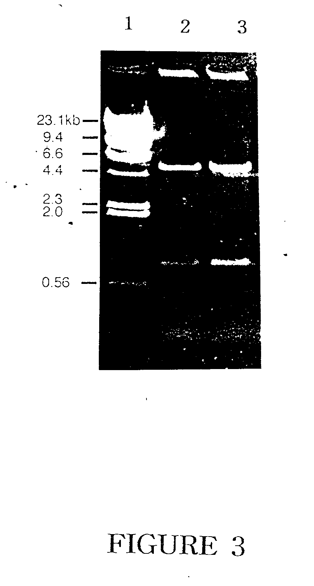

The recombinant expression vector thus prepared and competent E. coli JM101 were mixed, stood in ice for 60 minutes, incubated for 2 minutes at 42°C, and cultured for 1 hour at 37°C with the addition of LB medium(10g/L peptone, 5g/L yeast extract, 10g/L NaCl). Then, cells were spread on the solid LB media containing 100µg/ml ampicillin and 0.1mM IPTG, and cultured overnight, and colonies whose growth was completely inhibited were selected. Plasmid was isolated from the selected colonies by the modified alkali lysis method(see: Brush, D. et al., Mol. Cell Biol., 5:1307-1317(1985)), cleaved with HindIII, and confirmed to contain 1kb-DNA by agarose gel electrophoresis (see: Figure 3). Also, proper insertion of desired gPIP2 gene into a pFLAG-1™ vector was also investigated, based on the determination of nucleotide sequence of both ends of inserted DNA using NH₂-terminal primer(26-mer) and COOH-terminal primer(24-mer) as followings:
NH₂-terminal primer
5'-GTAGTATTGCCAAGACCGTTTATAAG-3'(SEQ ID No:7)
COOH-terminal primer
5'-GACATAGTCCGACTTTTAGAAGAG-3'(SEQ ID No:8)
In Figure 3, lane 1 shows λDNA digested with HindIII as molecular size marker; lane 2 shows Hind III-cleaved expression vector containing the gPIP2 gene; and, lane 3 shows Hind III-cleaved expression vector containing the gPIP50 gene which was constructed in Example 2-4.

Expression vector thus constructed was transformed into Epicurian coli XL1-Blue. Transformant thus prepared was designated Epicurian coli XL1-Blue MRF' gPIP2, and deposited under the Budapest Treaty with the Korean Culture Center of Microorganism(KCCM), an international depositary authority as deposition No. KCCM-10080 on April 10, 1996.

### Example 2: Isolation of gPIP50 gene from genomic library of Phytolacca insularis Nakai and construction of an expression vector therefor

DNA fragment containing the gPIP50 gene was isolated from genomic library derived from leaves of Phytolacca insularis Nakai using the cPIP gene labelled with [α-³²p]dATP as a probe. PCR was carried out by employing the prepared DNA fragment as a template to amplify specific region of the gPIP50 gene coding for mature protein, and the amplified DNA was inserted into a pFLAG-1™ vector to construct an expression vector of the gPIP50 protein.

### Example 2-1: Isolation of genomic DNA of Phytolacca insularis Nakai and construction of genomic library

To isolate genomic DNA from leaves of Phytolacca insularis Nakai, 10g of leaves was grinded under liquid nitrogen, incubated for 2 hours at 55°C with the addition of 30ml of DNA extraction buffer(100mM Tris-HCl(pH 8.0) containing 100mM EDTA, 250mM NaCl, and 100µg/ml proteinase K) and 10%(w/v) sarcosyl to a final concentration of 1%, and centrifuged at 5,500 x g for 10 minutes at 4°C to obtain supernatant. Isopropanol equivalent to 0.6 volume of the supernatant was added to the supernatant, and centrifuged at 7,500 x g for 10 minutes to obtain precipitate. Precipitate thus obtained was dissolved in 9ml of TE buffer, incubated for 30 minutes in ice with the addition of 9.7g of CsCl, and centrifuged at 7,500 x g for 10 minutes to obtain supernatant. 0.5ml of 1%(w/v) ethidium bromide was added to the supernatant, centrifuged analogously as the above to obtain supernatant, and ultracentrifugation of the supernatant was followed at 60,000rpm for 16 hours to precipitate DNA. Precipitated DNA was washed repeatedly with isopropanol saturated with CsCl to remove ethidium bromide, incubated for 1 hour at -20°C with the addition of distilled water and ethanol, and centrifuged at 7,500 x g for 10 minutes at 4°C to obtain precipitate. The precipitate was resuspended in TE buffer, and centrifuged as mentioned above with the addition of ethanol equivalent to two volume of the said resuspension to isolate genomic DNA from Phytolacca insularis Nakai.

To construct genomic library of Phytolacca insularis Nakai from the genomic DNA thus isolated, partial digestion of the genomic DNA with Sau3AI was carried out to obtain about 10kb-DNA fragments. 0.9µg of 10kb-DNA fragment thus obtained was ligated with 1.0µg of λGEM-11 BamHI arm(Promega, USA) by T4 DNA ligase, and in vitro packaging employing Packagene(Promega, USA) was followed.

### Example 2-2: Screening of the genomic library of Phytolacca insularis Nakai and isolation of gPIP50 gene

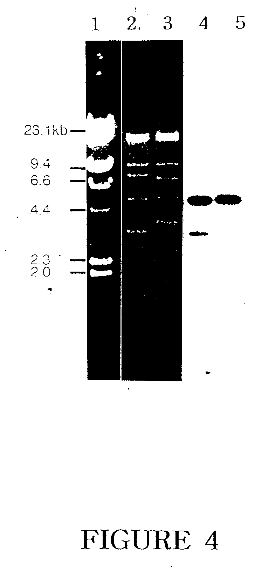

To select gPIP50 gene from the genomic library prepared in Example 2-1, 600,000 plaques in total were screened over three times of experiments where PCR product comprising the full coding region of cPIP labelled with (α-³²P]dATP by random primer labelling was used as a DNA probe, finally to give nine positive plaques. Each of nine plaques selected was cultured in LB medium employing E. coli KW251 as a host cell, and centrifugation was carried out to obtain supernatant. Supernatant thus obtained was ultracentrifuged at 25,000rpm for 2 hours to obtain phage particles. The phage particles thus obtained were treated with SDS, RNase and proteinase K, and phenol extraction were followed to purify phage DNA. Purified phage DNA was digested with SacI and XhoI, and analyzed by Southern hybridization using the DNA probe as mentioned above, which confirmed that there were clones which show positive signal of hybridization in XhoI-cleaved 5.0kb-DNA fragment and SacI-cleaved 4.8kb- and 3.5kb-DNA fragments. Among them, clone #50 showing strong positive signal was selected(see: Figure 4). In Figure 4, lane 1 shows λDNA digested with HindIII as molecular size marker; lanes 2 and 3 show 1% agarose gel electrophoresis pattern of phage DNA digested with SacI and XhoI, respectively; and, lanes 4 and 5 show corresponding Southern blot analysis of electrophoresis as shown in lanes 2 and 3, respectively.

5.0kb-DNA fragment of the selected clone #50 was subcloned on SalI-cleaved pUC19 vector, and the resulting plasmid was designated pPIP50. To prepare the restriction enzyme map of pPIP50, digestion of pPIP50 with BamHI, EcoRI, KpnI or SacI, and double-digestion with BamHI+HindIII or EcoRI+KpnI were carried out, and analyzed by Southern blot as mentioned above. The restriction enzyme map thus prepared revealed that only one band of 3.4kb-DNA fragment showed positive signal among BamHI fragments, and two bands did among EcoRI, KpnI, or SacI fragments, respectively. Accordingly, it was clearly demonstrated that 3.4kb-BamHI fragment contains the full gPIP50 gene hybridized with the said probe, and there are EcoRI, KpnI, and SacI restriction enzyme sites within the gene.

### Example 2-3: Determination of the nucleotide sequence of the gPIP50 gene

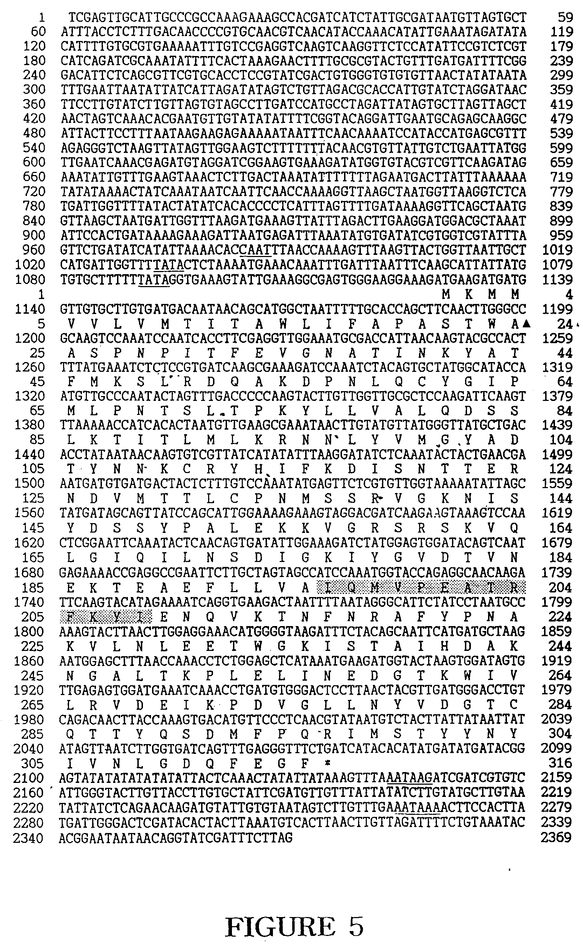

The nucleotide sequence of the gPIP50 gene located in 3.4kb-BamHI fragment which has confirmed in the restriction enzyme map of plasmid pPIP50, was determined by the conventional method in the art. From the nucleotide sequencing studies, it was revealed that the gPIP50 gene coding for the mature protein consists of 951bp including the start codon of translation, and its open reading frame encodes a 35.7kDa-protein which consists of 315 amino acids including initiation methionine(see: Figure 5). Further, it was also confirmed that 24 hydrophobic amino acids of amino-terminus play as a signal peptide. In Figure 5, a putative CAAT box and two TATA boxes commonly present in eukaryotes in 5'flanking region, and 3'-polyadenylation signal were underlined, (▲) indicates a putative cleavage site of signal peptide, shadow does amino acid sequence of a putative active site, and (*) does termination codon.

Based on the comparison of the amino acid sequence deduced from the said gPIP50 gene with ones from cPIP and α-PAP genes coding for known antiviral proteins, one from the gPIP50 gene was found to show 69.4% homology to one from cPIP gene, and 84% homology to one from α-PAP gene. In particular, it was determined that the amino acid sequence translated from the gPIP50 gene differs only in two residues of conserved site from known RIP proteins: that is, 200th and 203th amino acids of gPIP50 protein are proline and threonine, while their corresponding amino acids of cPAP or cPIP gene are mostly serine or alanine. However, it was suggested that the difference has little effect on the activity of RIP protein, since it is caused by the substitution of amino acid which is similar in terms of electrical property. Therefore, it was clearly demonstrated that the gPIP50 protein may have an antiviral activity very similar to one of the RIP proteins from the cPIP, α-PAP, and cPAP genes.

### Example 2-4: Construction of an expression vector containing the gPIP50 gene

To clone gPIP50 gene coding for the mature protein into an expression vector, pFLAG™, PCR was performed as described in Example 1-2 employing 3.4kb-BamHI fragment obtained in Example 2 as a template, and oligonucleotides with HindIII restriction enzyme sites at their both ends as primers as followings:
NH₂-terminal primer
5'-CCAAGCTTGCAAGTCCAAATCCAATC-3'(SEQ ID No:9)
COOH-terminal primer
5'-GGAAGCTTTGATCAGAAACCCTCAAA-3'(SEQ ID No:6)

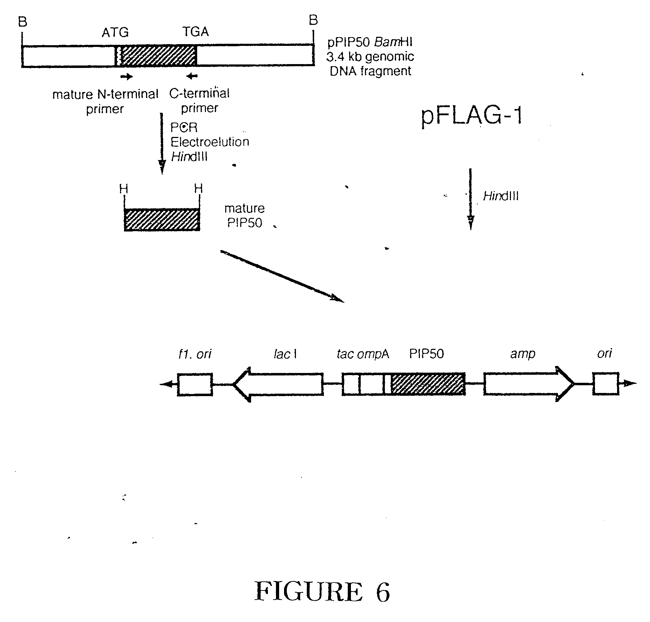

After 0.8% agarose gel electrophoresis of amplified PCR product, 1kb-DNA fragment containing the gPIP50 gene was electroeluted, digested with HindIII, and cloned on a HindIII-digested pFLAG-1™ vector(see: Figure 6). Figure 6 shows a construction strategy of an expression vector comprising a gPIP50 gene which is amplified by PCR using a 3.4kb-BamHI fragment derived from plasmid pPIP50.

E. coli was transformed with the recombinant expression vector thus prepared, and the resulting transformant was spread on a solid LB media containing 1.5mM IPTG as in Example 1-3, to select colonies whose growth was inhibited. Plasmids were isolated from the colonies thus selected, digested with HindIII, and analyzed by agarose gel electrophoresis to confirm 1kb-DNA band(see: Figure 3). Also, the nucleotide sequence of DNA insert cloned on pFLAG-1™ vector was determined using N-terminal primer(26-mer) and C-terminal primer(24-mer) in Example 1-3, which finally confirmed proper insertion of desired pPIP50 gene.

Expression vector thus constructed was transformed into Epicurian coli XL1-Blue. Transformant thus prepared was designated E. coli XL1-Blue MRF' gPIP50, and deposited under the Budapest Treaty with the Korean Culture Center of Microorganism(KCCM), an international depositary authority as deposition No. KCCM-10081 on April 10, 1996.

### Example 3: Expression of recombinant gPIP2 and gPIP50 proteins

E. coli transformed with the expression vector constructed in Examples 1-3 and 2-4, i.e., E. coli XL1-Blue MRF' gPIP2 and E. coli XL1-Blue MRF' gPIP50 were cultured in 500ml of LB medium at a shaking speed of 200rpm for 37°C to reach OD₆₀₀=0.7, and further cultured for 3 hours with the addition of IPTG(isopropyl-β-D-thiogalactoside) to a final concentration of 0.75mM in order to induce the desired recombinant protein. Cultured cells here harvested by centrifuging at 8,000rpm for 8 minutes at 4°C, resuspended in 35ml of PBS buffer(0.01M NaH₂PO₄, and 0.15M NaCl, pH 8.4), ultrasonicated, centrifuged at 15,000rpm for 20 minutes at 4°C, and collected supernatant and pellet separately. 1M CaCl₂ was added to the supernatant thus obtained(supernatant I) to a final concentration of 1.0mM, and to the pellet, was added 5ml of 8M urea, and centrifuged at 8,000rpm for 10 minutes to obtain supernatant. Supernatant thus obtained(supernatant II) was dialyzed against PBS buffer at 4°C overnight, and 1M CaCl₂ was added to supernatant II to a final concentration of 1.0mM.

Supernatant II containing CaCl₂ was loaded on anti-FLAG M1 affinity gel(International Biotechnologies Inc., USA) column which was washed three times with 5ml of glycine-HCl buffer(pH 3.0), and three times with 5ml of PBS buffer. Then, the column was washed three times with 12ml of PBS/Ca buffer(PBS buffer containing 1.0mM CaCl₂), and 0.5ml of PBS/EDTA(PBS buffer containing 2.0mM EDTA) was loaded onto the column, and stood for 30 minutes to chelate calcium ion, finally to give fractions of the pure recombinant protein by the intermittent elution with 0.5ml of PBS/EDTA at an interval of 10 minutes.

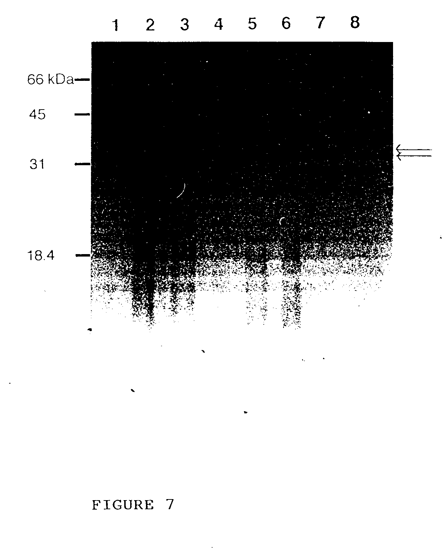

Based on 15% SDS-PAGE analysis of the said supernatants and the active fractions thus eluted, it was found that: about 33kDa-recombinant gPIP50 or gPIP2 protein is expressed successfully in the transformant, molecular weight of gPIP50 protein is somewhat larger than one of gPIP2 protein, and the expressed recombinant proteins form inclusion body, in light of a fact that most of them exist in supernatant II(see: Figure 7).

In Figure 7, lane 1 shows control supernatant I, i.e., supernatant I obtained from E. coli which was transformed with pFLAG-1™ vector and cultured under IPTG induction; lane 2 shows supernatant I obtained from E. coli XL1-Blue MRF' gPIP50; lane 3 shows supernatant I obtained from E. coli XL1-Blue MRF' gPIP2; lane 4 shows control supernatant II, i.e., supernatant II obtained from E. coli which was transformed with pFLAG-1™ vector and cultured under IPTG induction; lane 5 shows supernatant II obtained from E. coli XL1-Blue MRF' gPIP50; lane 6 shows supernatant II obtained from E. coli XL1-Blue MRF' gPIP2; lane 7 shows the recombinant gPIP50 protein purified from E. coli XL1-Blue MRF' gPIP50; lane 8 shows the recombinant gPIP2 protein purified from E. coli XL1-Blue MRF' gPIP2; and, arrows indicate the bands of the recombinant proteins.

### Example 4: Inhibition of protein synthesis by the recombinant gPIP2 or gPIP50 protein

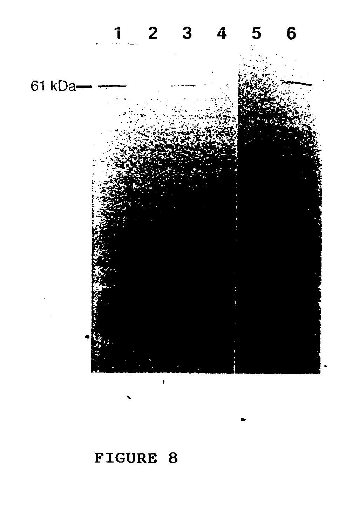

To study whether the recombinant gPIP2 or gPIP50 protein purified in Example 3 inhibits protein synthesis or not, in vitro translation assay was performed employing rabbit reticulocyte lysate system(Promega, USA): The recombinant protein was added to the reaction mixture containing luciferase mRNA(Promega, USA), rabbit reticulocyte lysate, amino acid mixture free of methionine, and ³⁵S-labelled methionine, and incubated for 90 minutes at 30°C. Proteins thus synthesized were separated on 15% SDS-PAGE, and the level of protein synthesis was assayed by autoradiography(see: Figure 8).

In Figure 8, lane 1 shows in vitro translation assay using 2.5µl of PBS/EDTA elution buffer in anti-FLAG M1 affinity gel chromatography as a substitute for the recombinant protein; lanes 2 and 3 show in vitro translation assay with the addition of the recombinant gPIP50 protein to a final concentration of 100pM and 10pM, respectively; lane 4 shows in vitro translation assay with the addition of the recombinant gPIP2 protein to a final concentration of 10pM; and, lanes 5 and 6 show in vitro translation assay using 400ng of supernatant II obtained from E. coli which was transformed with pFLAG-1™ vector and cultured under IPTG induction, without and with luciferase mRNA, respectively. While 61kDa-luciferase protein was synthesized with no addition of the recombinant gPIP50 or gPIP2 as shown in lane 1 and 6, no protein synthesis occurred with the addition of 100pM recombinant gPIP50 protein as shown in lane 2, and protein synthesis was markedly inhibited with the addition of 10pM recombinant gPIP50 or gPIP2 protein as shown in lane 3 and 4. Accordingly, it was confirmed that the purified recombinant gPIP2 or gPIP50 protein successfully inhibits protein synthesis.

As clearly illustrated and demonstrated above, the present invention provides novel gPIP2 and gPIP50 genes coding for antiviral proteins isolated from genomic DNA of Phytolacca insularis Nakai, gPIP2 and gPIP50 proteins having the amino acid sequences deduced from the said genes, respectively, expression vectors containing the said genes, and processes for preparing recombinant gPIP2 and gPIP50 proteins from recombinant microorganisms transformed with the said vectors. gPIP2 or gPIP50 gene encoding an antiviral protein can be employed in the manufacture of useful plants having antiviral resistance. Also, the recombinant gPIP2 or gPIP50 protein prepared in a microorganism transformed with an expression vector containing gPIP2 or gPIP50 gene can be used as an active ingredient of antiviral agents of plant viruses, and employed in the manufacture of immunoconjugates used for the treatment of AIDS and cancer.

The principal antiviral proteins of the invention have the amino acid sequences shows in SEQ ID NO: 2 (gPIP2) and SEQ ID NO: 4 (gPIP50). Other antiviral proteins may have functionally equivalent amino acid sequences. For example, the amino acid sequence shown in SEQ ID NO: 2 or 4 modified to contain one or more conservative amino acid substitutions constitutes a functionally equivalent amino acid sequence.

There may be from 1 to 20, for example from 1 to 15 or from 1 to 10, such conservative substitutions in the functionally equivalent amino acid sequence. Thus, there may be 1, 2, 3, 4 or 5 conservative substitutions. The following Table sets out conservative substitutions which may be made. Amino acids in the same line may be substituted for each other:

| Table of conservative amino acid substitutions | | |
|---|---|---|
| Gly | Ala | |
| Val | Ile | Leu |
| Asp | Glu | |
| Asn | Gln | |
| Ser | Thr | |
| Lys | Arg | |
| Phe | Tyr | |

Typically, a functionally equivalent amino acid sequence with respect to gPIP2 is an amino acid sequence which has at least 95% amino acid homology (identity) with the amino acid sequence of gPIP2. The homology may be at least 97%, at least 98% or at least 99%.

A functionally equivalent amino acid sequence with respect to gPIP50 is generally an amino acid sequence which has at least 90% amino acid homology (identity) with the amino acid sequence of gPIP50. The homology may be at least 95%, at least 97%, at least 98% or at least 99%.

An antiviral protein according to the invention which is functionally equivalent to gPIP2 or gPIP50 is capable of inhibiting protein synthesis in the *in vitro* translation system of Example 4. The functionally equivalent protein may contain amino acid deletions and/or insertions as well as or as an alternative to amino acid substitutions such as the conservative amino acid substitutions mentioned above.

The genes of the invention are typically DNA sequences. The gPIP2 gene can have the nucleotide sequence shown in SEQ ID NO: 1 or a functionally equivalent nucleotide sequence. Such a functionally equivalent nucleotide sequence is a nucleotide sequence which also encodes gPIP2, taking into account the degeneracy of the genetic code. A functionally equivalent nucleotide sequence as regards the gPIP50 gene is a nucleotide sequence which differs from the nucleotide sequence shown in SEQ ID NO: 3 but which also encodes gPIP50 thanks to the degeneracy of the genetic code.

A gene according to the invention is typically obtained is isolated form. It may be purified. It can be inserted into a suitable expression vector. Expression vectors of the invention thus consist essentially of a gene of the invention operably linked to a promoter. The expression vector is typically a plasmid, virus or phage vector provided with an origin of replication. Transcriptional and translational control elements are provided.

A host is transformed or transfected with the expression vector. The vector is generally chosen on the basis that it is compatible with the host into which it is to be transformed or transfected. The host may be a microbial host. Suitable host cells are bacterial, yeast, insect or mammalian cells. Plant cells may be transfected.

Once expression of the antiviral protein has been achieved, it can be purified. It can be obtained in substantially isolated form.

## Claims

1. A gene coding for (a) an antiviral protein which has the amino acid sequence shown in SEQ ID NO: 2 or 4 or (b) a functional equivalent of the said protein.

2. A gene according to claim 1 which has the nucleotide sequence shown in SEQ ID NO: 1 or a functionally equivalent nucleotide sequence.

3. A gene according to claim 1 which has the nucleotide sequence shown in SEQ ID NO: 3 or a functionally equivalent nucleotide sequence.

4. An expression vector which comprises a gene as claimed in any one of the preceding claims operably linked to a promoter.

5. A host transformed or transfected with a gene as claimed in any one of claims 1 to 3.

6. A host according to claim 5 which is selected from *E. coli* XL1-Blue MRF' gPIP2 (KCCM-10080) and *E. coli* XL1-Blue MRF' gPIP50 (KCCM-10081).

7. An antiviral protein which has the amino acid sequence shown in SEQ ID NO: 2 or 4 or a functionally equivalent amino acid sequence.

8. A process for preparing an antiviral protein as claimed in claim 7, which process comprises maintaining a host as claimed in claim 5 under such conditions that the said protein is expressed and purifying the resulting said protein.

9. A process according to claim 8 which comprises:
(i) culturing *E. coli* XL1-Blue MRF' gPIP2 (KCCM-10080) or *E. coli* XL1-Blue MRF' gPIP50 (KCCM-10081) and inducing the expression of the said protein by the aid of isopropyl-β-D-thiogalactoside;
(ii) harvesting and lysing the *E. coli* thus induced and obtaining inclusion bodies thus released; and
(iii) purifying the said protein from the inclusion bodies.

10. A purified recombinant antiviral protein according to claim 7.


SEQUENCE LISTING

(1) GENERAL INFORMATION:

(i) APPLICANT:
(A) NAME: JINRO LIMITED
(B) STREET: 1448-3, Seocho-Dong, Seocho-Ku
(C) CITY: Seoul
(D) STATE: not applicable
(E) COUNTRY: Korea
(F) POSTAL CODE (ZIP): 137-070
(G) TELEPHONE: 0331-289-6163
(H) TELEFAX: 0331-281-8368

(ii) TITLE OF INVENTION: NOVEL GENES ENCODING ANTIVIRAL
PROTEINS OF Phytolacca insularis
Nakai AND RECOMBINANT MICROORGANISMS
EXPRESSING THE SAME PROTEINS

(iii) NUMBER OF SEQUENCES: 9

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30
(EPO)

(2) INFORMATION FOR SEQ ID NO: 1:

(i) SEQUENCE CHARACTERISTICS:
(A) LENGTH: 882 base pairs
(B) TYPE: nucleic acid
(C) STRANDEDNESS: single
(D) TOPOLOGY: linear

(ii) MOLECULE TYPE: DNA(genomic)

(iii) HYPOTHETICAL: NO

(iv) ANTI-SENSE: NO

(vi) ORIGINAL SOURCE:
(A) ORGANISM: Phytolacca insularis Nakai

(vii) IMMEDIATE SOURCE:
(A) LIBRARY: genomic library from Phytolacca
insularis Nakai

(xi) SEQUENCE DESCRIPTION: SEQ ID NO: 1:

ATG GTG AAT ACC ATC ATC TAC AAT GTT GGA AGT ACC ACC ATT AGC AAA 48
Met Val Asn Thr Ile Ile Tyr Asn Val Gly Ser Thr Thr Ile Ser Lys
1               5                   10                  15

TAC GCC ACT TTT CTG GAT AAT CTT CGT AAT GAA GCA AAA GAT CCA AGT 96
Tyr Ala Thr Phe Leu Asp Asn Leu Arg Asn Glu Ala Lys Asp Pro Ser
            20                  25                  30

TTA AAA TGC TAT GGA ATA CCA ATG TTG CCC AAT ACA AAT CCA AAT CCA 144
Leu Lys Cys Tyr Gly Ile Pro Met Leu Pro Asn Thr Asn Pro Asn Pro
        35                  40                  45

AAG TAC GTG TTG GTT AAG CTC CAA GGT TCA AAT GAA AAA ACC ATC ACA 192
Lys Tyr Val Leu Val Lys Leu Gln Gly Ser Asn Glu Lys Thr Ile Thr
    50                  55                  60

CTA ATG CTG AGA CGA AAC AAT TTG TAT GTG ATG GGC TAT TCT GAT CCC 240
Leu Met Leu Arg Arg Asn Asn Leu Tyr Val Met Gly Tyr Ser Asp Pro
65                  70                  75                  80

TTT GAT ACC AAT AAG TGT CGT TAC CAT ATC TTT AAT GAT ATC TCA GGT 288
Phe Asp Thr Asn Lys Cys Arg Tyr His Ile Phe Asn Asp Ile Ser Gly
                85                  90                  95

ACT GAA CGC CAA GAT GTA GAG ACT ACT CTT TGC CCA AAT CCC AAT TCT 336
Thr Glu Arg Gln Asp Val Glu Thr Thr Leu Cys Pro Asn Pro Asn Ser
            100                 105                 110

CGT GTT AGT AAA AAC ATA AAC TAT GAT AGT CGA TAT CCA ACA TTG GAA 384
Arg Val Ser Lys Asn Ile Asn Tyr Asp Ser Arg Tyr Pro Thr Leu Glu
        115                 120                 125

TCA AAA GCG GGA GTA AAA TCA AGA AGT CAA GTT CAA CTG GGA ATT CAA 432
Ser Lys Ala Gly Val Lys Ser Arg Ser Gln Val Gln Leu Gly Ile Gln
    130                 135                 140

ATA CTC GAC AGT GAC ATT GGA AAG ATT TCT GGG GTG ACG TCA TTC ACT 480
Ile Leu Asp Ser Asp Ile Gly Lys Ile Ser Gly Val Thr Ser Phe Thr
145                 150                 155                 160

GAG AAA GTC GAA GCT GAA TTC CTA CTG GTA GCC ATA CAA ATG GTA TCA 528
Glu Lys Val Glu Ala Glu Phe Leu Leu Val Ala Ile Gln Met Val Ser
                165                 170                 175

GAG GCA GCA AGA TTC AAG TAC ATA GAG AAT CAG GTG AAA ACG AAT TTT 576
Glu Ala Ala Arg Phe Lys Tyr Ile Glu Asn Gln Val Lys Thr Asn Phe
            180                 185                 190

AAC AGA GCA TTC AAC CCT AAT CCC AAA GTA CTT AAT TTG GAA GAG ACA 624
Asn Arg Ala Phe Asn Pro Asn Pro Lys Val Leu Asn Leu Glu Glu Thr
        195                 200                 205

TGG GGT AAG ATT TCT ACA GCA ATT CAT GAT GCC AAG AAT GGA GTT TTA 672
Trp Gly Lys Ile Ser Thr Ala Ile His Asp Ala Lys Asn Gly Val Leu
    210                 215                 220

CCC AAA CCT CTC GAG TTA GTG GAT GCC AGT GGT GCC AAT TGG ATG GTG 720
Pro Lys Pro Leu Glu Leu Val Asp Ala Ser Gly Ala Asn Trp Ile Val
225                 230                 235                 240

TTG AGA GTG GAT GAT ATC AAG CCT GAT GTA GCA CTC TTA AAC TAC GTT 768
Leu Arg Val Asp Asp Ile Lys Pro Asp Val Ala Leu Leu Asn Tyr Val
                245                 250                 255

AGT GGG AGC TGC CAA ACA AAT TAT AAC CAA AAT GCC ATG TTT CCT CAA 816
Ser Gly Ser Cys Gln Thr Asn Tyr Asn Gln Asn Ala Met Phe Pro Gln
            260                 265                 270

CTT ATA ATG TCT ACT TAT TAT AAT TAT ATG GCT AAT CAT GGT GAT CAG 864
Leu Ile Met Ser Thr Tyr Tyr Asn Tyr Met Ala Asn His Gly Asp Gln
        275                 280                 285

TTT GAG GGT TTC TGA TCA 882
Phe Glu Gly Phe
    290

(2) INFORMATION FOR SEQ ID NO: 2:

(i) SEQUENCE CHARACTERISTICS:
(A) LENGTH: 292 amino acids
(B) TYPE: amino acid
(D) TOPOLOGY: unknown

(ii) MOLECULE TYPE: protein

(xi) SEQUENCE DESCRIPTION: SEQ ID NO: 2:

Met Val Asn Thr Ile Ile Tyr Asn Val Gly Ser Thr Thr Ile 14

Ser Lys Tyr Ala Thr Phe Leu Asp Asn Leu Arg Asn Glu Ala 28

Lys Asp Pro Ser Leu Lys Cys Tyr Gly Ile Pro Met Leu Pro 42

Asn Thr Asn Pro Asn Pro Lys Tyr Val Leu Val Lys Leu Gln 56

Gly Ser Asn Glu Lys Thr Ile Thr Leu Met Leu Arg Arg Asn 70

Asn Leu Tyr Val Met Gly Tyr Ser Asp Pro Phe Asp Thr Asn 84

Lys Cys Arg Tyr His Ile Phe Asn Asp Ile Ser Gly Thr Glu 98

Arg Gln Asp Val Glu Thr Thr Leu Cys Pro Asn Pro Asn Ser 112

Arg Val Ser Lys Asn Ile Asn Tyr Asp Ser Arg Tyr Pro Thr 126

Leu Glu Ser Lys Ala Gly Val Lys Ser Arg Ser Gln Val Gln 140

Leu Gly Ile Gln Ile Leu Asp Ser Asp Ile Gly Lys Ile Ser 154

Gly Val Thr Ser Phe Thr Glu Lys Val Glu Ala Glu Phe Leu 168

Leu Val Ala Ile Gln Met Val Ser Glu Ala Ala Arg Phe Lys 182

Tyr Ile Glu Asn Gln Val Lys Thr Asn Phe Asn Arg Ala Phe 196

Asn Pro Asn Pro Lys Val Leu Asn Leu Glu Glu Thr Trp Gly 210

Lys Ile Ser Thr Ala Ile His Asp Ala Lys Asn Gly Val Leu 224

Pro Lys Pro Leu Glu Leu Val Asp Ala Ser Gly Ala Asn Trp 238

Ile Val Leu Arg Val Asp Asp Ile Lys Pro Asp Val Ala Leu 252

Leu Asn Tyr Val Ser Gly Ser Cys Gln Thr Asn Tyr Asn Gln 266

Asn Ala Met Phe Pro Gln Leu Ile Met Ser Thr Tyr Tyr Asn 280

Tyr Met Ala Asn His Gly Asp Gln Phe Glu Gly Phe 292

(2) INFORMATION FOR SEQ ID NO: 3:

(i) SEQUENCE CHARACTERISTICS:
(A) LENGTH: 951 base pairs
(B) TYPE: nucleic acid
(C) STRANDEDNESS: single
(D) TOPOLOGY: linear

(ii) MOLECULE TYPE: DNA(genomic)

(iii) HYPOTHETICAL: NO

(iv) ANTI-SENSE: NO

(vi) ORIGINAL SOURCE:
(A) ORGANISM: Phytolacca insularis Nakai

(vii) IMMEDIATE SOURCE:
(A) LIBRARY: genomic library from Phytolacca
insularis Nakai

(xi) SEQUENCE DESCRIPTION: SEQ ID NO: 3:

ATG AAG ATG ATG GTT GTG CTT GTG ATG ACA ATA ACA GCA TGG CTA ATT 48
Met Lys Met Met Val Val Leu Val Met Thr Ile Thr Ala Trp Leu Ile
1               5                   10                  15

TTT GCA CCA GCT TCA ACT TGG GCC GCA AGT CCA AAT CCA ATC ACC TTC 96
Phe Ala Pro Ala Ser Thr Trp Ala Ala Ser Pro Asn Pro Ile Thr Phe
            20                  25                  30

GAG GTT GGA AAT GCG ACC ATT AAC AAG TAC GCC ACT TTT ATG AAA TCT 144
Glu Val Gly Asn Ala Thr Ile Asn Lys Tyr Ala Thr Phe Met Lys Ser
        35                  40                  45

CTC CGT GAT CAA GCG AAA GAT CCA AAT CTA CAG TGC TAT GGC ATA CCA 192
Leu Arg Asp Gln Ala Lys Asp Pro Asn Leu Gln Cys Tyr Gly Ile Pro
    50                  55                  60

ATG TTG CCC AAT ACT AGT TTG ACC CCC AAG TAC TTG TTG GTT GCG CTC 240
Met Leu Pro Asn Thr Ser Leu Thr Pro Lys Tyr Leu Leu Val Ala Leu
65                  70                  75                  80

CAA GAT TCA AGT TTA AAA ACC ATC ACA CTA ATG TTG AAG CGA AAT AAC 288
Gln Asp Ser Ser Leu Lys Thr Ile Thr Leu Met Leu Lys Arg Asn Asn
                85                  90                  95

TTG TAT GTT ATG GGT TAT GCT GAC ACC TAT AAT AAC AAG TGT CGT TAT 336
Leu Tyr Val Met Gly Tyr Ala Asp Thr Tyr Asn Asn Lys Cys Arg Tyr
            100                 105                 110

CAT ATA TTT AAG GAT ATC TCA AAT ACT ACT GAA CGA AAT GAT GTG ATG 384
His Ile Phe Lys Asp Ile Ser Asn Thr Thr Glu Arg Asn Asp Val Met
        115                 120                 125

ACT ACT CTT TGT CCA AAT ATG AGT TCT CGT GTT GGT AAA AAT ATT AGC 432
Thr Thr Leu Cys Pro Asn Met Ser Ser Arg Val Gly Lys Asn Ile Ser
    130                 135                 140

TAT GAT AGC AGT TAT CCA GCA TTG GAA AAG AAA GTA GGA CGA TCA AGA 480
Tyr Asp Ser Ser Tyr Pro Ala Leu Glu Lys Lys Val Gly Arg Ser Arg
145                 150                 155                 160

AGT AAA GTC CAA CTC GGA ATT CAA ATA CTC AAC AGT GAT ATT GGA AAG 528
Ser Lys Val Gln Leu Gly Ile Gln Ile Leu Asn Ser Asp Ile Gly Lys
                165                 170                 175

ATC TAT GGA GTG GAT ACA GTC AAT GAG AAA ACC GAG GCC GAA TTC TTG 576
Ile Tyr Gly Val Asp Thr Val Asn Glu Lys Thr Glu Ala Glu Phe Leu
            180                 185                 190

CTA GTA GCC ATC CAA ATG GTA CCA GAG GCA ACA AGA TTC AAG TAC ATA 624
Leu Val Ala Ile Gln Met Val Pro Glu Ala Thr Arg Phe Lys Tyr Ile
        195                 200                 205

GAA AAT CAG GTG AAG ACT AAT TTT AAT AGG GCA TTC TAT CCT AAT GCC 672
Glu Asn Gln Val Lys Thr Asn Phe Asn Arg Ala Phe Tyr Pro Asn Ala
    210                 215                 220

AAA GTA CTT AAC TTG GAG GAA ACA TGG GGT AAG ATT TCT ACA GCA ATT 720
Lys Val Leu Asn Leu Glu Glu Thr Trp Gly Lys Ile Ser Thr Ala Ile
225                 230                 235                 240

CAT GAT GCT AAG AAT GGA GCT TTA ACC AAA CCT CTG GAG CTC ATA AAT 768
His Asp Ala Lys Asn Gly Ala Leu Thr Lys Pro Leu Glu Leu Ile Asn
                245                 250                 255

GAA GAT GGT ACT AAG TGG ATA GTG TTG AGA GTG GAT GAA ATC AAA CCT 816
Glu Asp Gly Thr Lys Trp Ile Val Leu Arg Val Asp Glu Ile Lys Pro
            260                 265                 270

GAT GTG GGA CTC CTT AAC TAC GTT GAT GGG ACC TGT CAG ACA ACT TAC 864
Glu Val Gly Leu Leu Asn Tyr Val Glu Gly Thr Cys Gln Thr Thr Tyr
        275                 280                 285

CAA AGT GAC ATG TTC CCT CAA CGT ATA ATG TCT ACT TAT TAT AAT TAT 912
Gln Ser Glu Met Phe Pro Gln Arg Ile Met Ser Thr Tyr Tyr Asn Tyr
    290                 295                 300

ATA GTT AAT CTT GGT GAT CAG TTT GAG GGT TTC TGA TCA 951
Ile Val Asn Leu Gly Glu Gln Phe Glu Gly Phe
305                 310                 315

(2) INFORMATION FOR SEQ ID NO: 4:

(i) SEQUENCE CHARACTERISTICS:
(A) LENGTH: 315 amino acids
(B) TYPE: amino acid
(D) TOPOLOGY: unknown

(ii) MOLECULE TYPE: protein

(xi) SEQUENCE DESCRIPTION: SEQ ID NO: 4:

Met Lys Met Met Val Val Leu Val Met Thr Ile Thr Ala Trp 14

Leu Ile Phe Ala Pro Ala Ser Thr Trp Ala Ala Ser Pro Asn 28

Pro Ile Thr Phe Glu Val Gly Asn Ala Thr Ile Asn Lys Tyr 42

Ala Thr Phe Met Lys Ser Leu Arg Asp Gln Ala Lys Asp Pro 56

Asn Leu Gln Cys Tyr Gly Ile Pro Met Leu Pro Asn Thr Ser 70

Leu Thr Pro Lys Tyr Leu Leu Val Ala Leu Gln Asp Ser Ser 84

Leu Lys Thr Ile Thr Leu Met Leu Lys Arg Asn Asn Leu Tyr 98

Val Met Gly Tyr Ala Asp Thr Tyr Asn Asn Lys Cys Arg Tyr 112

His Ile Phe Lys Asp Ile Ser Asn Thr Thr Glu Arg Asn Asp 126

Val Met Thr Thr Leu Cys Pro Asn Met Ser Ser Arg Val Gly 140

Lys Asn Ile Ser Tyr Asp Ser Ser Tyr Pro Ala Leu Glu Lys 154

Lys Val Gly Arg Ser Arg Ser Lys Val Gln Leu Gly Ile Gln 168

Ile Leu Asn Ser Asp Ile Gly Lys Ile Tyr Gly Val Asp Thr 182

Val Asn Glu Lys Thr Glu Ala Glu Phe Leu Leu Val Ala Ile 196

Gln Met Val Pro Glu Ala Thr Arg Phe Lys Tyr Ile Glu Asn 210

Gln Val Lys Thr Asn Phe Asn Arg Ala Phe Tyr Pro Asn Ala 224

Lys Val Leu Asn Leu Glu Glu Thr Trp Gly Lys Ile Ser Thr 238

Ala Ile His Asp Ala Lys Asn Gly Ala Leu Thr Lys Pro Leu 252

Glu Leu Ile Asn Glu Asp Gly Thr Lys Trp Ile Val Leu Arg 266

Val Asp Glu Ile Lys Pro Glu Val Gly Leu Leu Asn Tyr Val 280

Glu Gly Thr Cys Gln Thr Thr Tyr Gln Ser Glu Met Phe Pro 294

Gln Arg Ile Met Ser Thr Tyr Tyr Asn Tyr Ile Val Asn Leu 308

Gly Glu Gln Phe Glu Gly Phe 315

(2) INFORMATION FOR SEQ ID NO: 5:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: genomic DNA to mRNA

(vii) IMMEDIATE SOURCE:
(B) CLONE: 5' PRIMER

(xi) SEQUENCE DESCRIPTION: SEQ ID NO: 5:

CCAAGCTTGT GAATACCATC ATCTAC 16

(2) INFORMATION FOR SEQ ID NO: 6:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: genomic DNA to mRNA

(vii) IMMEDIATE SOURCE:
(B) CLONE: 3' PCR PRIMER

(xi) SEQUENCE DESCRIPTION: SEQ ID NO: 6:

GGAAGCTTTG ATCAGAAACC CTCAAA 16

(2) INFORMATION FOR SEQ ID NO: 7:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: genomic DNA to mRNA

(vii) IMMEDIATE SOURCE:
(B) CLONE: 5′ PRIMER

(xi) SEQUENCE DESCRIPTION: SEQ ID NO: 7:

GTAGTATTGC CAAGACCGTT TATAAG 16

(2) INFORMATION FOR SEQ ID NO: 8:

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: genomic DNA to mRNA

(vii) IMMEDIATE SOURCE:
(B) CLONE: 3′ PRIMER

(xi) SEQUENCE DESCRIPTION: SEQ ID NO: 8:

GACATAGTCC GACTTTTAGA AGAG 14

(2) INFORMATION FOR SEQ ID NO: 9:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: genomic DNA to mRNA

(vii) IMMEDIATE SOURCE:
(B) CLONE: 5′ PRIMER

(xi) SEQUENCE DESCRIPTION: SEQ ID NO: 9:

CCAAGCTTGC AAGTCCAAAT CCAATC 16